Europäisches Patentamt

European Patent Office

Office européen des brevets

(11) Numéro de publication: **0 242 244 B1**

(12) **FASCICULE DE BREVET EUROPEEN**

(45) Date de publication de fascicule du brevet:
10.07.91

(51) Int. Cl.⁵: **A61K 33/10**, A61K 47/12,
A61K 47/42

(21) Numéro de dépôt: 87400511.9

(22) Date de dépôt: 09.03.87

(54) Composition pharmaceutique consistant en une suspension de carbonate de calcium.

(30) Priorité: 10.03.86 FR 8603355

(43) Date de publication de la demande:
21.10.87 Bulletin 87/43

(45) Mention de la délivrance du brevet:
10.07.91 Bulletin 91/28

(84) Etats contractants désignés:
AT BE CH DE ES FR GB GR IT LI LU NL SE

(56) Documents cités:
FR-A- 2 387 911

JOURNAL OF PHARMACEUTICAL SCIENCE,
vol. 69, no. 10, octobre 1980, pages
1209-1214, American Pharmaceutical Association; J.S. TEMPIO et al.: "Flocculation effect of xanthan gum in pharmaceutical suspensions"

CHEMICAL ABSTRACTS, vol. 85, no. 6, 9 août
1976, page 284, résumé no. 37165p, Columbus, Ohio, US; S.K. BAVEJA et al.: "Rheology
of dispersion systems containing Hemiadelphis polyspermus Nees seed mucilage" &
INDIAN J. PHARM. 1976, 38(2), 35-8

(73) Titulaire: **UNIVERSITE DE BORDEAUX II**
**146, rue Léo Saignat**
**F-33000 Bordeaux(FR)**

(72) Inventeur: **Aumonier, Pierre**
**51, route D'Ivrac**
**F-33310 Lormont(FR)**
Inventeur: **Guyot, Michel**
**44, Allée de Cante-Rane**
**F-33450 St.-Sulpice et Cameyrac(FR)**
Inventeur: **Merlet, Jean-Paul**
**3 Bis, rue Marceau**
**F-33440 Ambares(FR)**
Inventeur: **Pometan, Jean-Paul**
**40, Avenue de la Croix Le Taillan-Medoc**
**F-33320 Eysines(FR)**

(74) Mandataire: **Rinuy, Santarelli**
**14, avenue de la Grande Armée**
**F-75017 Paris(FR)**

EP 0 242 244 B1

CHEMICAL ABSTRACTS, vol. 86, no. 2, 10
janvier 1977, page 195, résumé no. 8592r,
Columbus, Ohio, US; M. NAKAGAKI et al.:
"Physicochemical studies on the sedimentation state of suspension particles. VI. The
effects of sodium polyacrylate on the aggregation and sedimentation state of griseofulvin and calcium carbonate" & YAKUGAKU
ZASSHI 1976, 96(9), 1127-36

## Description

La présente invention a pour objet une composition pharmaceutique consistant en une suspension de carbonate de calcium.

Le carbonate de calcium et déjà connu comme médicament pour corriger le déséquilibre ionique de certains malades présentant une insuffisance rénale chronique et notamment ceux qui sont soumis à un traitement par dialyse.

Toutefois l'assimilation du carbonate de calcium par l'organisme nécessite l'absorption de ce sel à des doses très élevées qui peuvent atteindre 6 à 12 g. par jour (cf. FOURNIER, Nouv. Presse Médicale 1977, P. 3533-3537). La prise de ce produit, qui se présente sous forme de paquets ou sachets dont le contenu est à délayer dans l'eau, sous forme de gélules de grande taille ou sous forme de cachets azymes, est mal acceptée par les patients par suite des difficultés de déglutition et du goût peu agréable.

L'invention a pour objet d'éviter ces inconvénients en proposant une nouvelle forme galénique du carbonate de calcium qui soit plus acceptable par le malade.

Cette nouvelle forme consiste en une suspension de particules de ce sel présentant une granulométrie inférieure à 250 et de préférence à 50 micromètres et en particulier dans la gamme de 1 à 10 micromètres dans un milieu liquide comme l'eau qui est susceptible d'amener rapidement le carbonate de calcium au niveau du duodénum, en évitant dans une certaine mesure sa réaction avec l'acidité gastrique et sa transformation en chlorure dans l'estomac.

La difficulté était de trouver une suspension stable qui contienne une quantité aussi élevée que possible de particules de carbonate de calcium dans un volume aussi faible que possible et qui présente une viscosité suffisamment basse pour permettre sa manipulation par flacon ou par dose unitaire liquide.

Ces diverses exigences quelque peu contradictoires ont été résolues selon l'invention par la combinaison d'un agent dispersant avec un agent peptisant.

L'agent dispersant qui permet d'eviter une sédimentation des particules de carbonate de calcium doit être choisi parmi les diverses variétés de gomme xanthane.

Par gomme xanthane, on entend les diverses variétés de gomme xanthane de qualité satisfaisant aux exigences de la monographie figurant à l'USP XXI (1985).

Par agent peptisant on entend, dans le cadre de la présente invention, un agent qui soit à la fois générateur de charges négatives pour protéger l'agent dispersant et capable de complexer les ions calcium $Ca^{++}$ de sorte qu'une faible partie du calcium soit à l'état soluble sans être à l'état ionisé.

Des agents peptisants préférés selon l'invention sont des sels d'acide carboxylique qui ont en outre la propriété d'amener le pH d'une suspension aqueuse de carbonate de calcium dans une gamme comprise entre 6 et 8 ; c'est le cas, en particulier, du citrate monosodique, dont l'ion citrique présente l'avantage d'être un constituant biochimique normal de l'organisme.

A ces deux constituants essentiels, il convient d'ajouter un agent conservateur, un agent édulcorant et un agent aromatisant.

L'agent conservateur doit présenter une activité antimicrobienne et antifongique et des exemples de tels agents sont les parahydroxybenzoates d'alkyle inférieur comme ceux de méthyle ou de propyle.

L'agent édulcorant qui a pour but d'améliorer le goût peut être soit un sirop édulcorant glucidique calorigène comme le saccharose ou à voie métabolique adaptée au diabète soit un édulcorant de synthèse non calorigène pour les malades atteints de troubles du métabolisme glucidique.

L'agent aromatisant destiné à améliorer la flaveur du médicament peut consister par exemple en une essence soluble d'orange ou tout autre agent améliorant l'arome.

Les proportions de ces différents constituants dans la composition selon l'invention sont entre 150 et 250 g. de particules de carbonate de calcium de granulométrie inférieure à 250 micromètres, entre 6 et 10 g. d'agent dispersant, entre 1 et 3 g. d'agent peptisant et, éventuellement, une quantité d'agent conservateur correspondant aux limites imposées par la pharmacopée Européenne, une proportion d'agent édulcorant qui est de l'ordre de 100 à 200 g. et une quantité de l'ordre de 0,5 à 2 g. d'agent aromatisant, le reste de la suspension étant constitué par de l'eau déminéralisée pour compléter la composition jusqu'à une teneur de 1 litre.

Un exemple particulier de composition présentant une excellente stabilité depuis plus de six mois est le suivant :

| Carbonate de calcium (<50 micromètres) | 200 g. |
| Gomme xanthane | 8 g. |
| Citrate monosodique | 2 g. |
| Parahydroxybenzoate de méthyle | 1,4 g. |
| Sirop simple | 150 ml. |
| Essence soluble d'orange | 1 ml. |
| Eau déminéralisée    Q. S.P | 1 litre |

**Revendications**

1. Composition pharmaceutique consistant en une suspension de carbonate de calcium en milieu aqueux caractérisée en ce que les particules de carbonate de calcium présentant une granulométrie inférieure à 250 micromètres sont maintenues en suspension par la combinaison d'un agent dispersant choisi parmi les diverses variétés de gomme xanthane avec un agent peptisant, c'est-à-dire un agent qui est à la fois générateur de charges négatives pour protéger l'agent dispersant et complexant des ions calcium formés, cet agent étant de préférence un sel d'acide carboxylique ayant la propriété d'amener le pH de la suspension aqueuse du carbonate de calcium dans une gamme comprise entre 6 et 8, ledit agent étant en particulier le citrate monosodique.

2. Composition selon la revendication 1 caractérisée en ce qu'elle contient en outre un agent conservateur qui est de préférence un parahydroxybenzoate d'alkyle inférieur.

3. Composition selon l'une quelconque des revendications précédentes caractérisée en ce qu'elle contient également un agent éducorant, et notamment du saccharose.

4. Composition selon l'une quelconque des revendications précédentes caractérisée en ce qu'elle contient un agent aromatisant.

5. Composition selon l'une quelconque des revendications précédentes caractérisée en ce qu'elle contient entre 150 et 250 g; des dites particules de carbonate de calcium de granulométrie inférieure à 250 micromètres, entre 6 et 10 g. de gomme xanthane et entre 1 et 3 g. de citrate monosodique pour 1 litre de composition.

6. Composition selon la revendication 5, caractérisée en ce qu'elle contient en outre environ 150 ml. de sirop de saccharose, environ 1,4 g. de parahydroxybenzoate de méthyle et environ 1 ml. d'essence soluble d'orange.

**Claims**

1. A pharmaceutical composition consisting of a calcium carbonate suspension in an aqueous medium, characterised in that calcium carbonate particles having a particle size less than 250 micrometres are maintained in suspension by a combination of a dispersing agent, chosen from various types of xanthan gum and a peptizing agent, that is to say an agent which both produces negative charges for protecting the dispersing agent and forms complexes with calcium ions formed, the peptizing agent preferably being a carboxylic acid salt, which is capable of bringing the pH of the aqueous suspension of calcium carbonate into a range between 6 and 8, the said agent preferably being monosodium citrate.

2. A composition according to claim 1, characterised in that it also contains a preservative agent which is preferably a lower alkyl parahydroxybenzoate.

3. A composition according to any one of the preceding claims, characterised in that it also contains a sweetening agent, preferably sucrose.

4

EP 0 242 244 B1

4. A composition according to any one of the preceding claims, characterized in that it contains an aroma-imparting agent.

5. A composition according to any one of the preceding claims, characterized in that it contains between 150 and 250 g of calcium carbonate particles having a particle size less than 250 micrometres, between 6 and 10 9 of xanthan gum and between 1 and 3 g of monosodium citrate per litre of composition.

6. A composition according to claim 5, characterized in that it also contains about 150 ml of sucrose syrup, about 1.4 g of methyl parahydroxybenzoate and about 1 ml of soluble orange oil.

**Ansprüche**

1. Pharmazeutische Zusammensetzung bestehend aus einer wässerigen Calciumcarbonatsuspension, dadurch gekennzeichnet, daß die Calciumcarbonatteilchen mit einer Korngröße von weniger als 250 µm durch eine Kombination eines Dispergiermittels ausgewählt aus den verschiedenen Formen von Xanthangummi mit einem Peptisator, d.h. einem Mittel, das gleichzeitig ein Erzeuger von negativen Ladungen zum Schützen des Dispergiermittels und ein Komplexbildner der gebildeten Calciumionen ist, welches Mittel vorzugsweise ein Carbonsäuresalz ist, das die Eigenschaft hat, den pH der wässerigen Calciumcarbonatsuspension in einen Bereich von 6 bis 8 zu bringen, insbesondere Mononatriumcitrat, in Suspension gehalten werden.

2. Zusammensetzung nach Anspruch 1, dadurch gekennzeichnet, daß sie außerdem ein Konservierungsmittel enthält, das vorzugsweise ein nied.Alkyl-p-hydroxybenzoat ist.

3. Zusammensetzung nach einem der vorhergehenden Ansprüche, dadurch gekennzeichnet, daß sie auch einen Süßstoff, insbesondere Saccharose, enthält.

4. Zusammensetzung nach einem der vorhergehenden Ansprüche, dadurch gekennzeichnet, daß sie ein Aromatisierungsmittel enthält.

5. Zusammensetzung nach einem der vorhergehenden Ansprüche, dadurch gekennzeichnet, daß sie 150 bis 250 g Calciumcarbonatteilchen mit einer Korngröße von weniger als 250 µm, 6 bis 10 g Xanthangummi und 1 bis 3 g Mononatriumcitrat pro 1 Liter Zusammensetzung enthält.

6. Zusammensetzung nach Anspruch 5, dadurch gekennzeichnet, daß sie außerdem etwa 150 ml Saccharosesirup, etwa 1,4 g Methyl-p-hydroxybenzoat und etwa 1 ml lösliches Orangenöl enthält.

5